# EUROPEAN PATENT APPLICATION

(11) **EP 4 659 746 A1**
(43) Date of publication of application: **10.12.2025**
(21) Application number: 24750581.1
(22) Date of filing: 01.02.2024
(51) Int. Cl.: A61K 9/28, A23P 20/10

(54) **FILM COATING COMPOSITION THAT DOES NOT USE TITANIUM OXIDE SO AS TO BE SAFE, AND FOOD AND MEDICINE FORMULATIONS COATED THEREWITH**

(30) Priority: 02.02.2023 KR 20230014288
(71) Applicant: P2kbio Inc., Cheongju-si, Chungcheongbuk-do 28160 (KR)
(72) Inventor: PARK, Yun Sang, Yongin-si Gyeonggi-do 16848 (KR); PARK, Chun Woong, Sejong 30063 (KR); KIM, Dong Wook, Seoul 07694 (KR); KANG, Ji Hyun, Cheongju-si Chungcheongbuk-do 28160 (KR); NA, Sang Beom, Cheongju-si Chungcheongbuk-do 28162 (KR); HA, Yong Soo, Uijeongbu-si Gyeonggi-do 11718 (KR)
(74) Representative: Louis Pöhlau Lohrentz
(86) International application number: PCT/KR2024/001517
(87) International publication number: WO 2024/162784

(57) **Abstract**

The present invention relates to a film coating composition that does not use titanium oxide so as to be safe from genotoxicity, and food and medicine formulations coated therewith.

## Description

### TECHNICAL FIELD

The present invention relates to a film coating composition for coating food and medicine formulations and to food and medicine formulations coated therewith. In particular, the present invention may provide a film coating composition that does not use titanium oxide, which is an opacifying agent, so as to be safe from genotoxicity.

### BACKGROUND ART

Titanium dioxide is a white compound obtained by a reaction of titanium, a transition metal element, with oxygen. Since it has low reactivity and is very stable chemically, it is widely used as an additive in paints, dyes, foods, and medicines and also as an ingredient in UV blockers. When titanium dioxide is added to a film coating composition, it exhibits a high whiteness (ΔL), and accordingly, it plays a crucial role in the identification of medicines, that is, identification of medicines by color, which is important for the safe use of medicines. In addition, titanium dioxide has a high light-blocking effect, so it enables medicines to maintain stability from external light.

The safety of titanium dioxide was brought into question in 2017 by the French National Institute for Agricultural Research, which reported that titanium dioxide increased the risk of earlystage carcinogenesis of colon cancer in 40% of animals. Therefore, the European Food Safety Authority (EFSA) announced that it was withdrawing its approval for the use of titanium dioxide as a food additive. The EFSA cited the carcinogenicity of titanium dioxide due to its genotoxicity as the cause and stated that although the absorption of titanium dioxide particles is low after oral ingestion, it may accumulate in the body, and that considering scientific research and data, titanium dioxide is no longer safe as a food additive (https:/www.efsa.europa.eu/en/news/titaniumdioxide-e171-no-longer-considered-safe-when-used-food-additive).

However, titanium dioxide is widely used as a colorant in film coating base materials not only in foods but also in medicines, but due to the concerns about a shortage of medicines that may be caused by the ban on the use of titanium dioxide, the European Union (EU) postponed the ban on the use of titanium dioxide in medicines and announced that it will make a decision on the ban three years later (https:/www.ema.europa.eu/en/documents/report/final-feedback-european-medicine-agency-ema-eu-commission-request-evaluate-impact-removal-titanium_en.pdf).

As described above, the problem of carcinogenicity due to genotoxicity of titanium oxide, which is widely used in the food and medicinal fields, continues to be raised, and there is a need for the invention of a material or composition that can replace titanium oxide.

### [Related Art Documents]

### [Non-patent Documents]

(Non-patent Document 1) https:/www.efsa.europa.eu/en/news/titanium-dioxide-e171-no-longer-considered-safe-when-used-food-additive
(Non-patent Document 2) https:/www.ema.europa.eu/en/documents/report/final-feedback-european-medicine-agency-ema-eu-commission-request-evaluate-impact-removal-titanium_en.pdf

### DETAILED DESCRIPTION OF THE INVENTION

### TECHNICAL PROBLEM

The problem to be solved by the present invention is to provide a film coating composition for film coating of food and medicine formulations that exhibits high whiteness (ΔL) and light-blocking properties, which are the main functions of titanium oxide, without using titanium oxide, which is a genotoxic substance, and a medicine formulation and a food formulation to which the film coating composition is applied.

In particular, the film coating composition of the present invention does not have the genotoxicity problem of titanium oxide because it does not use titanium oxide, and it also has the characteristic of exhibiting whiteness (ΔL) and light-blocking properties similar to those of titanium oxide, without affecting the disintegration of uncoated tablets.

### TECHNICAL SOLUTION

The present inventors have found that a silicon dioxide metal salt is an additive that may be used in food and medicine formulations and added to a film coating composition as a component to smoothly form a film and is capable of blocking the color of uncoated tablets due to its high whiteness (ΔL) and exhibiting high light-blocking properties without significantly affecting the disintegration of uncoated tablets.

Conventionally, a silicon dioxide metal salt is a substance widely used as an additive for food and medicine formulations and has been used mainly for the purpose of improving the fluidity of powder or increasing the lubricating properties, and there has been no report on its use as a substitute for titanium oxide by adding it to a film coating composition, and the carcinogenicity of a silicon dioxide metal salt has not been reported.

A film coating composition prepared by adding a silicon dioxide metal salt as a substitute for titanium oxide exhibited a high whiteness (ΔL) and light-blocking properties even when titanium oxide was not used, and compared to titanium oxide, it did not further delay the disintegration time of uncoated tablets.

In most aspects of the present invention, a silicon dioxide metal salt exhibited a whiteness (ΔL) sufficient to replace titanium oxide, and when film coating was performed on a tablet using the film coating composition of the present invention, it showed improved storage stability compared to titanium oxide.

In an aspect of the present invention, there is provided a film coating composition comprising a coating polymer, a silicon dioxide metal salt, a plasticizer, and a solvent, and the film coating composition does not comprise titanium oxide. At this time, a surfactant or a colorant may be included according to the purpose.

### ADVANTAGEOUS EFFECTS

The present invention provides a film coating composition for food and medicine formulations, which is safe from genotoxicity because it does not use titanium oxide and exhibits high whiteness (ΔL) and light-blocking properties when filmcoated on formulations, which are the main characteristics of titanium oxide.

The effects of the present invention are not limited to the above-mentioned effects, and various effects may be included within a range that is obvious to those skilled in the art from the contents described below.

### DESCRIPTION OF THE DRAWINGS

The attached drawings illustrate preferred embodiments and should not be interpreted as being limited to the matters described in the drawings.

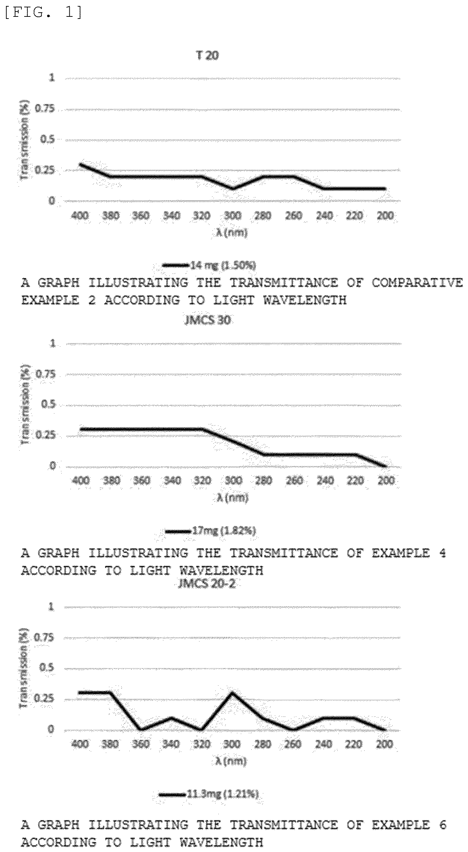

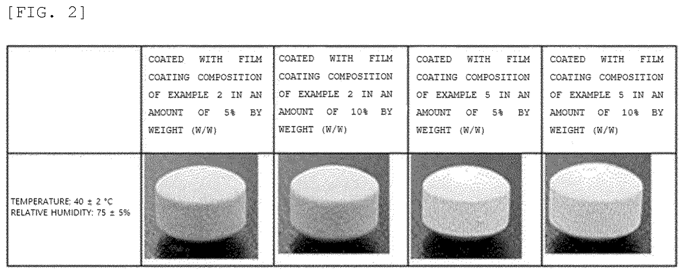

FIG. 1 shows a graph illustrating the light transmittance evaluation of the film coating composition of Comparative Example 2 containing 20% by weight of titanium oxide and the film coating compositions of Examples 4 and 6 containing 20% by weight of silicon dioxide metal salt, among film coating compositions. FIG. 2 shows the results of an accelerated stability evaluation conducted for 30 days under accelerated conditions (temperature: 40 ± 2 °C, relative humidity: 75 ± 5%).

### BEST MODE

Hereinafter, the present specification will be described in more detail.

The present specification is described in more detail as follows. The terms used herein are selected as general terms that are currently widely used as much as possible while considering the functions in the present invention, but they may vary depending on the intention or precedent of those of ordinary skill in the art, the emergence of new technology, or the like. In addition, in certain cases, there are terms arbitrarily selected by the applicant, and in this case, the meaning will be described in the corresponding part of the detailed description of the invention. Therefore, the terms used in the present invention should be defined based on the meaning of the terms and the overall content of the present invention, rather than simply the names of the terms.

Unless otherwise defined, all terms, comprising technical and scientific terms used herein, have the same meaning as generally understood by one of ordinary skill in the art to which the present invention pertains. Terms, such as those defined in commonly used dictionaries, should be interpreted as having a meaning that is consistent with their meaning in the context of the relevant art and are not interpreted in an idealized or overly formal sense unless clearly so defined in the present invention.

Numerical ranges are inclusive of the values defined therein. Every maximum numerical limitation given throughout the present specification includes every lower numerical limitation, as if such lower numerical limitations were expressly written. Every minimum numerical limitation given throughout the present specification includes every higher numerical limitation, as if such higher numerical limitations were expressly written. Every numerical limitation given throughout the present specification will include every better numerical range within the broader numerical range, as if the narrower numerical limitations were expressly written.

Hereinafter, each description and embodiment disclosed in the present invention may also be applied to other descriptions and embodiments for each. In other words, all combinations of various elements disclosed in the present invention fall within the scope of the present invention. In addition, the scope of the present invention may not be considered as being limited by the specific description described below.

Expressions such as "comprising" as used herein should be understood as open-ended terms implying the possibility of comprising other embodiments.

The present inventors screened substances widely used as additives in foods and medicines as a substitute for titanium oxide and confirmed that silicon dioxide metal salt had high whiteness (ΔL) and light-blocking properties and did not further delay the disintegration time of tablets compared to titanium oxide. Accordingly, after performing film coating on tablets, it was confirmed that the tablets exhibited improved storage stability compared to titanium oxide, thereby completing the present invention.

Hereinafter, the present invention is described in detail.

Unless otherwise specified herein, the term '% by weight' refers to the mass ratio of a particular component to the entire film coating composition into which it is incorporated.

### Film coating composition

In order to solve the above-described problems, the present invention discloses the following means.

In one aspect, the present invention discloses a film coating composition comprising: a coating polymer; a silicon dioxide metal salt; and a plasticizer, wherein the film coating composition does not comprise titanium oxide.

In the present invention, the content of the coating polymer may be 30% to 90% by weight based on the total weight of the entire film coating composition, specifically it may be 50% to 75% by weight, but is not limited thereto. When the content of the coating polymer is less than 30% by weight, there is a problem that the film is not formed due to insufficient coating polymer, and when it exceeds 90% by weight, there is a problem that the film layer breaks due to insufficient elasticity of the film layer due to low plasticizer content.

In the present invention, the content of the silicon dioxide metal salt may be 5% to 50% by weight based on the total weight of the entire film coating composition, specifically it may be 5% to 30% by weight, but is not limited thereto. When the content of the silicon dioxide metal salt is less than 5% by weight, there is a problem that light-blocking power or whiteness is not sufficient, and when it exceeds 50% by weight, there is a problem that a film is not formed due to insufficient coating polymer.

In the present invention, the content of the plasticizer may be 5% to 40% by weight based on the total weight of the entire film coating composition, specifically it may be 5% to 30% by weight, but is not limited thereto. When the content of the plasticizer is less than 5% by weight, there is a problem that the film layer has insufficient elasticity and thus the film layer breaks, and when it exceeds 40% by weight, there is a problem that the film layer has too high elasticity and thus its adhesiveness to tablets is low and the film formation is not smooth.

In the present invention, the film coating composition may be in the form of solid powder but is not limited thereto.

In the present invention, the film coating composition may be mixed with a solvent so that the content of the film coating composition is 5% to 30% by weight based on a final film coating solution but is not limited thereto. Specifically, the content of the film coating composition may be 5% to 30% by weight (w/w), specifically 10% to 25% by weight (w/w) based on the total amount of the solvent and the film coating composition used when dissolving the film coating composition in the solvent, but is not limited thereto. When the content of the film coating composition is less than 5% by weight, the content of the film coating composition contained in the solvent is too small, which causes a problem in that the film coating process takes a long time, and when it exceeds 30% by weight, the content of the film coating composition contained in the solvent is too high, which causes a problem in that the coating liquid may not be smoothly sprayed from a coating machine due to the high viscosity caused by the coating polymer.

In the present invention, the solvent may be selected from the group consisting of purified water, ethanol, and a mixture thereof but is not limited thereto.

In the present invention, the coating polymer may be either a non-pH dependent polymer or a pH dependent polymer but is not limited thereto.

In the present invention, the term "non-pH dependent polymer" refers to a polymer whose dissolution does not significantly change according to a change in pH. Specifically, the non-pH dependent polymer may be at least one selected from the group consisting of hydroxypropylmethyl cellulose, polyvinyl alcohol, polyvinyl alcohol-polyethylene glycol-graft-copolymer, and hydroxypropyl cellulose but is not limited thereto.

In the present invention, the term "pH dependent polymer" refers to a polymer whose dissolution changes according to a change in pH. Specifically, the pH-dependent polymer may be at least one selected from the group consisting of polyvinyl acetate phthalate, hydroxypropylmethyl cellulose acetate succinate, hydroxypropylmethyl cellulose phthalate, cellulose acetate phthalate, and a methacrylic acid copolymer but is not limited thereto.

In the present invention, the term "silicon dioxide metal salt" refers to an opacifying agent that functions to replace titanium oxide, and an opacifying agent refers to a substance added for the purpose of reducing the transparency of food or medicine. Specifically, the silicon dioxide metal salt may be at least one selected from the group consisting of calcium silicate, calcium silicate hydrate, sodium silicate, magnesium silicate, magnesium trisilicate, aluminium silicate, magnesium aluminometasilicate, potassium aluminium disilicate, and sodium aluminosilicate, and a silicon dioxide metal salt, which is a titanium oxide substitute used in a preferred embodiment of the present invention, may be at least one selected from the group consisting of calcium silicate, calcium silicate hydrate, sodium silicate, magnesium silicate, aluminum silicate, and magnesium aluminometasilicate but is not limited thereto.

In the present invention, the film coating composition may further comprise, in addition to the coating polymer and the silicon dioxide metal salt, a plasticizer for imparting elasticity to a film layer, a colorant for imparting color to the film layer, and a surfactant for increasing wettability of the film layer.

Hereinafter, a plasticizer, a colorant, and a surfactant are described. At this time, a plasticizer is included as an essential component in the film coating composition, and a colorant and a surfactant may be included according to the purpose (need) .

In the present invention, the plasticizer may be at least one selected from the group consisting of triethyl citrate, tributyl citrate, glyceryl triacetate, acetyl triethyl citrate, dibutyl sebacate, diethyl phthalate, polyethylene glycol having a molecular weight ranging from 200 to 20,000, castor oil, and a copolymer of propylene oxide and ethylene oxide, and a plasticizer used in a preferred embodiment of the present invention may be at least one selected from the group consisting of triethyl citrate, tributyl citrate, glyceryl triacetate, acetyl triethyl citrate, diethyl phthalate, and polyethylene glycol having a molecular weight ranging from 200 to 20,000, but is not limited thereto.

In the present invention, the colorant may comprise an aluminum lake-based colorant, for example, but is not limited thereto.

In the present invention, the surfactant may comprise polysorbate, sodium lauryl sulfate, and the like but is not limited thereto.

In the present invention, the content of each of the colorant and the surfactant may be 0% to 15% by weight based on the total weight of the entire film coating composition, and preferably may be 0% to 10% by weight, but is not limited thereto. Here, 0% by weight means that it is not included in the film coating composition, and a lower limit may be expressed as 0% by weight. In other words, as described above, in the present invention, the content of the colorant and the surfactant being 0% by weight means that they may be used optionally according to the need.

In the present invention, the film coating composition may be for film coating applied to a medicine formulation but is not limited thereto.

In the present invention, the film coating composition may be for film coating applied to a food formulation but is not limited thereto.

### Medicine formulation

In another aspect, the present invention discloses a medicine formulation in which a medicine is coated with the film coating composition.

Specifically, the film coating composition comprises a medicine formulation that forms a film coating by being applied to the surface of the medicine formulation. In this case, the film coating composition may be applied to the medicine formulation through a general process as part of a pan coating or spray coating process. The coating amount of the applied film coating composition is 1% to 30% by weight (w/w), preferably 2% to 20% by weight (w/w), and more preferably 2% to 10% by weight (w/w), based on the weight of an uncoated medicine formulation. For coating applied to a formulation having multiple particles (active ingredients themselves in pellets or fine particles), it is required that the coating composition should be applied at a substantially higher percentage by weight because the surface area of the formulation is large. In this case, the coating amount of the film coating composition is 5% to 50% by weight (w/w) based on the weight of an uncoated medicine formulation, and preferably 10% to 40% by weight (w/w).

The above descriptions of the film coating composition may be applied to all medicine formulations in which a medicine is coated with the film coating composition, unless they are contradictory.

### Food formulation

In another aspect, the present invention discloses a food formulation in which a food is coated with the film coating composition.

Specifically, the film coating composition comprises a food formulation that is applied to the surface of a food to form a film coating. In this case, the film coating composition may be applied to the food formulation through a general process as part of a pan coating or spray coating process. The coating amount of the applied film coating composition is 1% to 30% by weight (w/w) based on the weight of an uncoated food formulation, preferably 2% to 20% by weight (w/w), and more preferably 2% to 10% by weight (w/w) .

The above descriptions of the film coating composition may be applied to all food formulations in which a food is coated with the film coating composition, unless they are contradictory.

Hereinafter, the present invention will be described in detail based on examples. However, the following examples are only intended to illustrate the present invention, and the scope of the present invention is not limited thereto.

### Examples and Comparative Examples

### Comparative Examples 1 and 2. Film coating compositions

A film coating composition of Comparative Example 1, in which titanium oxide or a titanium oxide substitute was not used, and a film coating composition of Comparative Example 2, in which titanium oxide was used, were prepared with the compositions and contents shown in Table 1 below.

Specifically, a coating base material (hydroxypropylmethyl cellulose) and a plasticizer (polyethylene glycol 400) were well mixed to prepare a film coating composition according to Comparative Example 1, and a coating base material (hydroxypropylmethyl cellulose), a plasticizer (polyethylene glycol 400), and an opacifying agent (titanium oxide) were well mixed to prepare a film coating composition according to Comparative Example 2.

**[Table 1]**

| Mixing purpose | Name of raw material | Comparative Example 1 | Comparative Example 2 |
|---|---|---|---|
| | | Content Percentage (% by weight) | Content Percentage (% by weight) |
| Coating base material | Hydroxypropylmethyl cellulose | 90 | 73 |
| Opacifying agent | Titanium oxide | - | 20 |
| Plasticizer | Polyethylene glycol 400 | 10 | 7 |
| Total | | 100 | 100 |

(In Table 1 above, the content percentage refers to percentage by weight.)

### Examples

### Examples 1-6. Film coating compositions containing calcium silicate

Film coating compositions containing calcium silicate, which is a silicon dioxide metal salt, were prepared with the compositions and contents shown in Table 2 below.

Specifically, a coating base material (hydroxypropylmethyl cellulose), a plasticizer (polyethylene glycol 400 or polyethylene glycol 6000), and an opacifying agent (calcium silicate) were well mixed to prepare the film coating compositions according to Examples 1 to 6.

**[Table 2]**

| Mixing purpose | Name of raw material | Example 1 | Example 2 | Example 3 | Example 4 | Example 5 | Example 6 |
|---|---|---|---|---|---|---|---|
| | | Content Percent age (% by weight) | Content Percent age (% by weight) | Content Percent age (% by weight) | Content Percent age (% by weight) | Content Percent age (% by weight) | Content Percent age (% by weight) |
| Coating base material | Hydroxypropyl methyl cellulose | 73 | 78 | 68 | 58 | 58 | 53 |
| Opacify ing agent | Calcium silicate | 20 | 10 | 20 | 30 | 20 | 20 |
| Plastic izer | Polyethylene glycol 400 | 7 | - | - | - | - | - |
| | Polyethylene glycol 6000 | - | 12 | 12 | 12 | 22 | 27 |
| Total | | 100 | 100 | 100 | 100 | 100 | 100 |

(In Table 2 above, the content percentage refers to percentage by weight.)

### Examples 7 to 13. Film coating compositions containing various non-pH dependent coating base materials, silicon dioxide metal salts, and plasticizers

Non-pH dependent film coating compositions containing silicon dioxide metal salts such as calcium silicate, sodium silicate, magnesium silicate, or magnesium aluminometasilicate were prepared with the compositions and contents shown in Table 3 below.

Specifically, a non-pH dependent coating base material (hydroxypropylmethyl cellulose, polyvinyl alcohol, or polyvinyl alcohol-polyethylene glycol-graft-copolymer), a plasticizer (polyethylene glycol 6000, triethyl citrate, or glyceryl triacetate), and an opacifying agent (calcium silicate, sodium silicate, magnesium silicate, or magnesium aluminometasilicate) were well mixed to prepare film coating compositions according to Examples 7 to 13.

**[Table 3]**

| Mixing purpose | Name of raw material | Example 7 | Example 8 | Example 9 | Example 10 | Example 11 | Example 12 | Example 13 |
|---|---|---|---|---|---|---|---|---|
| | | Content Percen tage | Content Percen tage | Content Percen tage | Content Percen tage | Content Percen tage | Content Percen tage | Content Percen tage |
| | | (% by weight ) | (% by weight ) | (% by weight ) | (% by weight ) | (% by weight ) | (% by weight ) | (% by weight ) |
| Coating base material | Hydroxyprop ylmethyl cellulose | 68 | 68 | - | - | - | - | - |
| | Polyvinyl alcohol | - | - | 58 | - | 58 | 58 | 58 |
| | Polyvinyl alcohol-polyethylene glycol-graft-copolymer | - | - | - | 58 | - | - | - |
| Opacif ying agent | Calcium silicate | 20 | 20 | 20 | 20 | - | - | - |
| | Sodium silicate | - | - | - | - | 20 | - | - |
| | Magnesium silicate | - | - | - | - | - | 20 | - |
| | Magnesium aluminometa silicate | - | - | - | - | - | - | 20 |
| Plasti cizer | Polyethylene glycol 6000 | - | - | 22 | 22 | 22 | 22 | 22 |
| | Triethyl citrate | 12 | - | - | - | - | - | - |
| | Glyceryl triacetate | - | 12 | - | - | - | - | - |
| Total | | 100 | 100 | 100 | 100 | 100 | 100 | 100 |

(In Table 3 above, the content percentage refers to percentage by weight.)

### Examples 14 to 16. Film coating compositions containing various pH-dependent coating base materials, calcium silicate, and plasticizers

pH-dependent film coating compositions containing calcium silicate which is a silicon dioxide metal salt were prepared with the compositions and contents shown in Table 4 below.

Specifically, a pH-dependent coating base material (hydroxypropylmethyl cellulose phthalate, hydroxypropylmethyl cellulose acetate succinate, or polyvinyl acetate phthalate), a plasticizer (triethyl citrate), and an opacifying agent (calcium silicate) were well mixed to prepare pH-dependent film coating compositions according to Examples 14 to 16.

**[Table 4]**

| Mixing purpose | Name of raw material | Example 14 | Example 15 | Example 16 |
|---|---|---|---|---|
| | | Content Percentage (% by weight) | Content Percentage (% by weight) | Content Percentage (% by weight) |
| Coating base material | Hydroxypropylmethyl cellulose phthalate | 70 | - | - |
| | Hydroxypropylmethyl cellulose acetate succinate | - | 70 | - |
| | polyvinyl acetate phthalate | - | - | 70 |
| Opacifying agent | Calcium silicate | 5 | 5 | 5 |
| Plasticizer | Triethyl citrate | 25 | 25 | 25 |
| Total | | 100 | 100 | 100 |

(In Table 4 above, the content percentage refers to percentage by weight.)

Hereinafter, the present invention will be described in detail based on experimental examples. However, the following experimental examples are only for illustrating the present invention, and the scope of the present invention is not limited thereto.

### Preparation Examples

### Preparation of uncoated tablets

For the evaluation of whiteness (ΔL), disintegration, and stability, red tablets containing a red colorant were prepared with the composition shown in Table 5 below.

Specifically, microcrystalline cellulose, lactose hydrate, and crospovidone were mixed. Povidone was dissolved in purified water, and then red iron oxide was dispersed, and then the resulting mixture was added to the above-described mixture to knead. The kneaded product was dried and then sieved. Magnesium stearate was added to the sieved product and finally mixed. The final mixture was pressed into tablets using a rotary tablet press with an 8 mm round punch to prepare red tablets.

**[Table 5]**

| Mixing purpose | Name of raw material | Content percentage (% by weight) |
|---|---|---|
| Excipient | Microcrystalline cellulose | 60 |
| Excipient | Lactose hydrate | 30 |
| Binder | Povidone | 3 |
| Disintegrant | Crospovidone | 5 |
| Colorant | Red iron oxide | 1 |
| Activator | Magnesium stearate | 1 |
| Total | | 100 |

(In Table 5 above, the content percentage refers to percentage by weight.)

### Coating conditions

When coating the prepared uncoated tablets using the film coating compositions according to the comparative examples and the examples in the following experimental examples, the coating conditions are as follows.
(1) Coating conditions using purified water as a solvent (Examples 1 to 13)
   Supply air temperature: 55 to 60 °C
   Exhaust air temperature: 40 to 45 °C
   Coating pan rotation speed: 5 to 10 rpm
   Film coating solution: The film coating composition of Examples 1 to 13 is dissolved or dispersed in purified water so that the concentration of the film coating solution becomes 10% (w/w) .
(2) Coating conditions using 80% ethanol aqueous solution (v/v) as a solvent (Examples 14 to 16)
   Supply air temperature: 45 to 50 °C
   Exhaust air temperature: 35 to 40 °C
   Coating pan rotation speed: 5 to 10 rpm
   Film coating solution: The film coating composition of Examples 14 to 16 is dissolved or dispersed in an 80% ethanol aqueous solution (v/v) so that the concentration of the film coating solution becomes 10% (w/w).

### Experimental Examples

### Experimental Example 1. Carcinogenicity evaluation

### Evaluation method

To evaluate the genotoxicity and carcinogenicity of calcium silicate-a representative silicon dioxide metal salt among various types-assessments were performed following the ICH M7 guideline. This guideline focuses on DNA reactive substances that can directly damage DNA, potentially causing mutations and leading to cancer. Genotoxicity was evaluated using the computer programs Dreck Ver. 6.2.1. and Sarah Ver. 3.2.1., based on Quantitative Structure-Activity Relationship ((Q)SAR) assessment, and carcinogenicity was also evaluated through a literature search. The results are presented in Table 6.

For the literature search, reference was made to Cited Documents 1 to 3.

Cited document 1: Safety assessment of titanium dioxide (E171) as a food additive. EFSA journal 2021.

Cited document 2: European chemical substrate information system.

Cited document 3: Re-evaluation of calcium silicate (E 552), magnesium silicate (E 553a(i)), magnesium trisilicate (E 553a(ii)) and talc (E 553b) as food additives. EFSA journal 2018.

### Evaluation results

Referring to Table 6, genotoxicity was not confirmed for titanium oxide or calcium silicate, a representative silicon dioxide metal salt, by QSAR evaluation. However, while the carcinogenicity of titanium oxide was reported in the literature, no carcinogenicity was confirmed for calcium silicate in literature reports.

**[Table 6]**

| Evaluated substance | (Q) SAR Derek | (Q) SAR Sarah | Carcinogenicity literature |
|---|---|---|---|
| Titanium dioxide | Inactive | Negative | Carcinogenicity reported¹⁾²⁾ |
| Calcium silicate | Inactive | Negative | Carcinogenicity not reported³⁾ |

(In Table 6, 1) refers to cited documents 1 and 2, refers to 2 and 3) refers to cited document 3.)

### Experimental Example 2. Evaluation of whiteness (ΔL)

### Evaluation method

Using the film coating compositions of Comparative Examples 1 and 2 and Examples 2 to 6, coating was performed on the red uncoated tablets prepared according to Preparation Examples by adjusting the coating amount based on the weight of the uncoated tablets. (At this time, the coating amount (w/w) of the film coating composition based on the weight of the uncoated tablets was set to 0%, 3%, 5%, and 10%. Here, 0% may be considered to mean the uncoated tablets themselves.)

Specifically, a whiteness (ΔL) evaluation was performed using a colorimeter on tablets coated with a white film coating composition using red uncoated tablets, and the whiteness (ΔL), which is the difference of white compared to the standard white, was evaluated. Regarding the whiteness (ΔL), a difference of 10 compared to the standard white is a difference that may not be easily determined with the naked eye, and this was used as a criterion for judgment. The results are shown in Table 7.

### Evaluation results

Referring to Table 7, a clear color difference shift from red to white was confirmed for the film coating compositions according to Examples 2 to 6 containing a silicon dioxide metal salt when compared to Comparative Example 1.

In addition, compared to the film coating composition containing titanium oxide (Comparative Example 2) which is prohibited for use in food due to genotoxicity issues, in the case of the film coating compositions according to Examples 2 to 6 containing a silicon dioxide metal salt applied in an amount of about 3% to 10% by weight (w/w) based on the weight of the uncoated tablet, it was confirmed that a similar level of whiteness (ΔL) could be obtained as the film coating composition containing titanium oxide applied in an amount of 3% by weight (w/w) based on the weight of the uncoated tablet (Comparative Example 2 composition). Therefore, it was confirmed that the film coating compositions according to Examples 2 to 6 were suitable as white film coating compositions.

**[Table 7]**

| Classificatio n | Comparativ e Example 1 | Comparativ e Example 2 | | Exampl e 2 | Exampl e 3 | Exampl e 4 | Exampl e 5 | Exampl e 6 |
|---|---|---|---|---|---|---|---|---|
| Type of opacifying agent | - | Titanium oxide | | Silicon dioxide metal salt (calcium silicate) | | | | |
| Amount of opacifying agent used | | - | 20% | 10% | 20% | 30% | 20% | 20% |
| Amount of coating based on the weight of uncoated tablets (w/w) | 0% | 27.2 | 27.2 | 27.1 | 27.3 | 29.7 | 27.0 | 29.0 |
| | 3% | 25.8 | 7.55 | 22.6 | 19.1 | 11.1 | 10.1 | 10.1 |
| | 5% | 25 | 3.09 | 20.6 | 16.5 | 8.1 | 8.3 | 8.2 |
| | 10% | 24.2 | 1.84 | 16.6 | 12.9 | 7.3 | 7.4 | 6.2 |

(In Table 7 above, the unit of the amount of opacifying agent used refers to percentage by weight.)

### Experimental Example 3. Evaluation of light-blocking properties (transmittance)

### Evaluation method

A light-blocking property (transmittance) evaluation was performed using the film coating compositions of Comparative Example 2 and Examples 4 and 6.

Specifically, for the light-blocking properties (transmittance) evaluation, the quartz cell of an absorbance meter was coated with the film coating compositions of Comparative Example 2 and Examples 4 and 6. The light-blocking properties (transmittance) of the film layers were evaluated by measuring the degree of transmission at various wavelengths. The results are shown in Table 8 and FIG. 1. (In Table 8, the coating amount (%, w/w) was determined by converting the film layer thickness obtained in Example 1. That is, the film layer thickness was set based on the weight of the film layer coated on the uncoated tablets. For instance, when coating uncoated tablets at 3% (w/w), the thickness of the film layer formed on the quartz cell was measured. If this measured thickness is set to 100, then coating at 1.5% (w/w) implies coating the quartz cell to form a film layer with a thickness of 50, representing half the thickness of the film layer coated at 3% (w/w).)

### Evaluation results

Referring to Table 8 and FIG. 1, it was confirmed that the film coating compositions of Examples 4 and 6 according to the present invention showed a transmittance of less than 0.5% even with a coating amount of less than 2% by weight (w/w) based on the weight of uncoated tablets, compared to the film coating composition of Comparative Example 2 in which titanium oxide was used. Through this, it was confirmed that sufficient light-blocking properties (transmittance) was obtained by applying the film coating compositions of Examples 4 and 6 according to the present invention.

**[Table 8]**

| Comparative Example 2 | | Example 4 | | Example 6 | |
|---|---|---|---|---|---|
| Containing 20% by weight of titanium oxide | | Containing 30% by weight of calcium silicate | | Containing 30% by weight of calcium silicate | |

| Amount of coating (w/w) | Light transmittance | Amount of coating (w/w) | Light transmittance | Amount of coating (w/w) | Light transmittance |
|---|---|---|---|---|---|
| 1.50% | Less than 0.5% | 1.82% | Less than 0.5% | 1.21% | Less than 0.5% |

### Experimental Example 4. Evaluation of disintegration

### Evaluation method

A disintegration evaluation was performed using the film coating compositions of Comparative Example 2 and Examples 4 and 6.

Specifically, in order to evaluate the disintegration, the disintegration time delay was evaluated according to the coating amount based the weight of the uncoated tablets prepared according to Preparation Example (The coating amount (w/w) of the film coating composition based on the weight of the uncoated tablets was set to 0%, 3%, 5%, and 10%. Here, 0% may be considered to mean the uncoated tablets themselves.) in accordance with the disintegration test method among the general test methods described in the Korean Pharmacopoeia, and the results are shown in Table 9.

### Evaluation results

Referring to Table 9, the evaluation results showed there was a difference of at least 1.5 minutes and at most 2.5 minutes compared to the disintegration time of the uncoated tablets, which was about 3.5 minutes, and the disintegration time was similar to the disintegration time of Comparative Example.

**[Table 9]**

| Classification | | Comparative Example 1 | Comparative Example 2 | Example 2 | Example 3 | Example 5 |
|---|---|---|---|---|---|---|
| Type of opacifying agent | | - | Titanium oxide | Silicon dioxide metal salt (calcium silicate) | | |
| Amount of opacifying agent used | | - | 20% | 10% | 20% | 20% |
| Disintegration time of uncoated tablets | | About 3.5 minutes | | | | |
| Amount of coating based on the weight of uncoated tablets (w/w) | 3% | About 1 minute | About 1 minute | About 5 minutes | About 5 minutes | About 5.5 minutes |
| | 5% | About 1 minute | About 1 minute | About 5.5 minutes | About 5 minutes | About 5 minutes |
| | 10% | About 3 minutes | About 2 minutes | About 6 minutes | About 5.5 minutes | About 5.5 minutes |

(In Table 9 above, the unit of the amount of opacifying agent used refers to percentage by weight.)

### Experimental Example 5. Storage stability evaluation

### Evaluation method

An accelerated stability evaluation was conducted using the film coating compositions of Examples 2 and 5.

Specifically, for a stability test, the red uncoated tablets prepared according to Preparation Example were coated with the film coating compositions of Examples 2 and 5 in a coating amount (w/w) of 5% by weight (w/w) and 10% by weight (w/w) based on the weight of the uncoated tablets, respectively (refer to the above-described coating method), and the coated tablets were placed in a stability test chamber. An accelerated stability evaluation was conducted for 30 days under accelerated conditions (temperature: 40 ± 2 °C, relative humidity: 75 ± 5%), and the results are shown in Fig. 2.

### Evaluation results

Referring to Fig. 2, the evaluation results confirmed that in the cases in which the coating amount of the film coating compositions according to Examples 2 and 5 was 5% by weight (w/w) and 10% by weight (w/w) of the weight of the uncoated tablets prepared according to Preparation Example, the coating applied on the surface of the tablets was not damaged after 30 days from the test start date.

Therefore, it was confirmed that the film coating composition according to the present invention has excellent storage stability.

As described above, specific parts of the present invention have been described in detail, and it is obvious that these specific descriptions are only preferred embodiments for those skilled in the art and that the scope of the present invention is not limited thereto.

Accordingly, the substantial scope of the present invention will be defined by the appended claims and their equivalents.

## Claims

1. A film coating composition comprising: a coating polymer; a silicon dioxide metal salt; and a plasticizer, wherein the film coating composition does not comprise titanium oxide.

2. The film coating composition according to claim 1, wherein the content of the coating polymer is 30% to 90% by weight based on the total weight of the entire film coating composition.

3. The film coating composition according to claim 1, wherein the content of the silicon dioxide metal salt is 5% to 50% by weight based on the total weight of the entire film coating composition.

4. The film coating composition according to claim 1, wherein the content of the plasticizer is 5% to 40% by weight based on the total weight of the entire film coating composition.

5. The film coating composition according to claim 1, wherein the film coating composition is in the form of solid powder.

6. The film coating composition according to claim 1, wherein the film coating composition is mixed with a solvent so that the content of the film coating composition is 5% to 30% by weight based on a final film coating solution.

7. The film coating composition according to claim 1, wherein the coating polymer is either a non-pH dependent polymer or a pH dependent polymer.

8. The film coating composition according to claim 7, wherein the non-pH dependent polymer is at least one selected from the group consisting of hydroxypropylmethyl cellulose, polyvinyl alcohol, polyvinyl alcohol-polyethylene glycol-graft-copolymer, and hydroxypropyl cellulose.

9. The film coating composition according to claim 7, wherein the pH-dependent polymer is at least one selected from the group consisting of polyvinyl acetate phthalate, hydroxypropylmethyl cellulose acetate succinate, hydroxypropylmethyl cellulose phthalate, cellulose acetate phthalate, and a methacrylic acid copolymer.

10. The film coating composition according to claim 1, wherein the silicon dioxide metal salt is at least one selected from the group consisting of calcium silicate, calcium silicate hydrate, sodium silicate, magnesium silicate, magnesium trisilicate, aluminium silicate, magnesium aluminometasilicate, potassium aluminium disilicate, and sodium aluminosilicate.

11. The film coating composition according to claim 1, wherein the plasticizer is at least one selected from the group consisting of triethyl citrate, tributyl citrate, glyceryl triacetate, acetyl triethyl citrate, dibutyl sebacate, diethyl phthalate, polyethylene glycol having a molecular weight ranging from 200 to 20,000, castor oil, and a copolymer of propylene oxide and ethylene oxide.

12. The film coating composition according to claim 1, wherein the film coating composition is for film coating applied to a medicine formulation.

13. The film coating composition according to claim 1, wherein the film coating composition is for film coating applied to a food formulation.

14. A medicine formulation in which a medicine is coated with the film coating composition according to claim 1.

15. A food formulation in which a food is coated with the film coating composition according to claim 1.
